# EUROPEAN PATENT SPECIFICATION

(11) **EP 1 272 245 B1**
(45) Date of publication and mention of the grant of the patent: **19.07.2006**
(21) Application number: 01919641.9
(22) Date of filing: 10.04.2001
(51) Int. Cl.: A61M 16/08, A61M 39/26

(54) **SELF SEALING WATER TRAP**
SELBSTDICHTENDE WASSERFALLE
SEPARATEUR D'EAU AUTO-OBTURANT

(30) Priority: 14.04.2000 GB 0009205
(43) Date of publication of application: 08.01.2003
(73) Proprietor: INTERSURGICAL LIMITED, Wokingham Berkshire RG41 2RZ (GB)
(72) Inventor: JASSELL, Surinderjit, Kumar, Middlesex UB8 6DE (GB)
(74) Representative: Jones, Stephen Anthony
(86) International application number: PCT/GB2001/001598
(87) International publication number: WO 2001/078819

(56) References cited:
- EP-A- 0 460 731
- EP-A- 0 672 430
- EP-A- 0 705 616
- GB-A- 2 272 745
- US-A- 4 417 574

## Description

This invention relates to a self-sealing water trap, and in particular to such a trap which is of utility in systems for the mechanical ventilation of a patient incapable of spontaneous respiration.

In a healthy person the function of breathing is entirely spontaneous. The brain senses a build-up of carbon dioxide in the blood and immediately calls for more oxygen. This oxygen is taken into the body by spontaneous inspiration and carbon dioxide is removed in the passive exhalation phase of respiration. A healthy person generates a certain amount of humidity, which is used in the lung to stop the build-up of secretions.

The ability to breathe spontaneously may be lost for a number of reasons.
Examples are as a result of surgical procedures (post-operatively), as a result of certain muscular disorders affecting the lung, or as a result of sedation by a clinician. Patients thus affected must be ventilated by mechanical means in order to achieve oxygenation and carbon dioxide removal.

When a patient is mechanically ventilated it is essential that the humidity of the air is maintained at a sufficiently high level, since a lung with impaired function will be more susceptible to secretions. This can be achieved using a heat-moisture exchanger (HME) or a heated water bath humidifier. An HME retains the moisture in an exhaled breath and this moisture is sent back to the lung with the next inspiratory phase. In a water bath system the inspiratory gas is passed through a heated water chamber and picks up humidity prior to entering the lung. As the humid inspiratory gas travels along the breathing system a certain amount of water vapour will cool and start to condense, forming water droplets, which will start to build up, causing so-called "rain-out".

Such water has to be removed from the breathing system so that it does not occlude the respiratory air flow or drain back into the patient's lungs thereby putting the patient at risk of drowning, or does not drain into the ventilator/anaesthetic equipment thus causing damage. If it is allowed to accumulate for a protracted period then due to its non-compressible nature the water will effectively block the breathing system.

The most effective way of collecting moisture in such a system is by the use of a device called a water trap. Such a device is generally located at the mid-point of the breathing system and positioned at the lowest point so that liquid will drain into it. It is necessary periodically to empty accumulated water from such a water trap and this should ideally be possible without interrupting ventilation of the patient and also without causing a drop in the pressure within the system, and without permitting escape of possibly contaminated or infectious fluid from the system or infection of the system by external agents.

EP-A1-0 705 616 discloses the features of the preamble of claim 1.

There therefore exists a need for a water trap which satisfies these requirements and the present invention addresses that need.

According to the invention there is provided a water trap suitable for use in a mechanical ventilation circuit, the water trap comprising a trap top having formed therein an inlet and an outlet in fluid communication via a chamber, and having a circular base with at least one base aperture formed therein, a rotatable valve plate in abutting relation to said base and rotatable relative thereto, said valve plate having at least one valve aperture adapted by rotation of said valve plate to be brought into or out of registration with said base aperture, and a fluid collection vessel releasable from the rest of the water trap and operably linked to said valve plate such that, when said fluid collection vessel is engaged with the rest of the water trap, said base aperture and said valve aperture are in registration and said fluid collection vessel is in fluid communication with said trap top and, when said fluid collection vessel is removed from the rest of the water trap, said base aperture and said valve aperture are not in registration and said fluid collection vessel is isolated from said trap top,
wherein the base is moulded in rigid plastics material and an elastomeric seal is subsequently moulded to the surface of said base that abuts said valve plate, the elastomeric seal comprising a ring of elastomeric material around the at least one base aperture.

The water trap according to the invention is advantageous primarily in that removal of the collection vessel from the trap, eg for emptying, automatically closes the at least one base aperture from the fluid collection vessel, and reengagement of the collection vessel subsequently automatically reopens the base aperture to the fluid collection vessel. This eliminates or substantially mitigates disadvantages associated with the prior art, such as interruption of ventilation, a reduction in internal pressure in the system, leakage of fluid from the system or contamination of the system from outside.

The fluid collection vessel is preferably operably linked to the rotatable valve plate such that the valve plate is rotated by rotation of the collection vessel, such rotation also releasing the collection vessel from the rest of the trap.

The collection vessel most preferably has a quick release type connection to the rest of the trap, eg a bayonet-type fitting or similar. A screw-threaded connection may also be possible.

According to another aspect of the invention there is provided a mechanical ventilation circuit including a water trap as described above.

A currently preferred embodiment of the invention will now be described in greater detail, by way of example only, with reference to the accompanying drawings, in which
Figure 1 is a perspective view of a self sealing water trap according to the invention;
Figure 2 is a view similar to Figure 1, partially cut away;
Figure 3 is cross-sectional side view of the water trap, with an internal seal in an open condition;
Figure 4 is a view similar to Figure 3 with the internal seal closed and a collecting cup disengaged;
Figure 5 is an exploded view of the water trap;
Figure 6 is an inverted exploded view of the water trap;
Figure 7 is a perspective view of the water trap with the top partially cut away; and
Figure 8 is a view of the water trap from above.

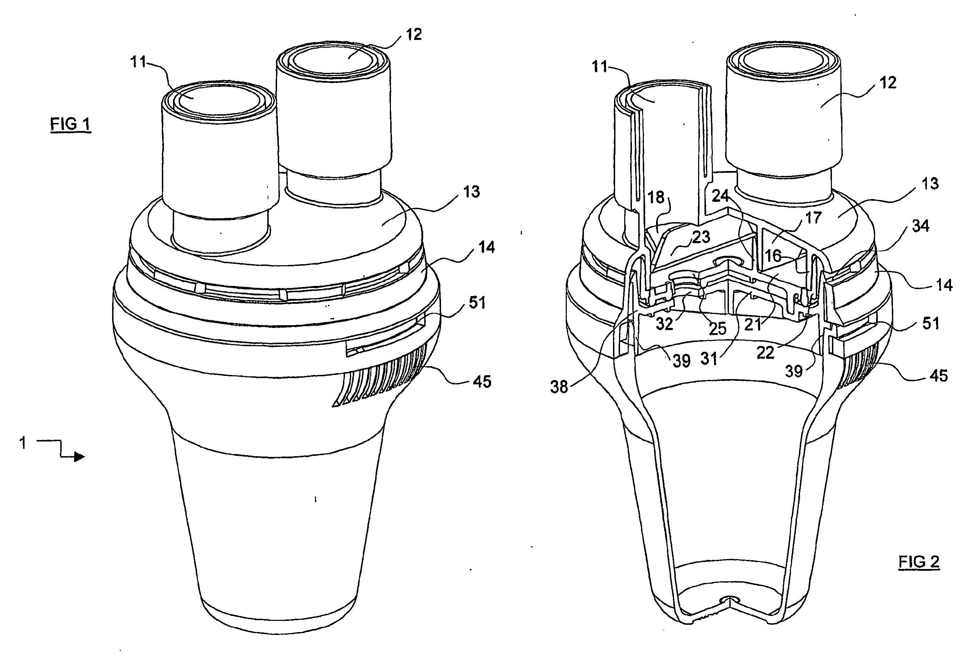

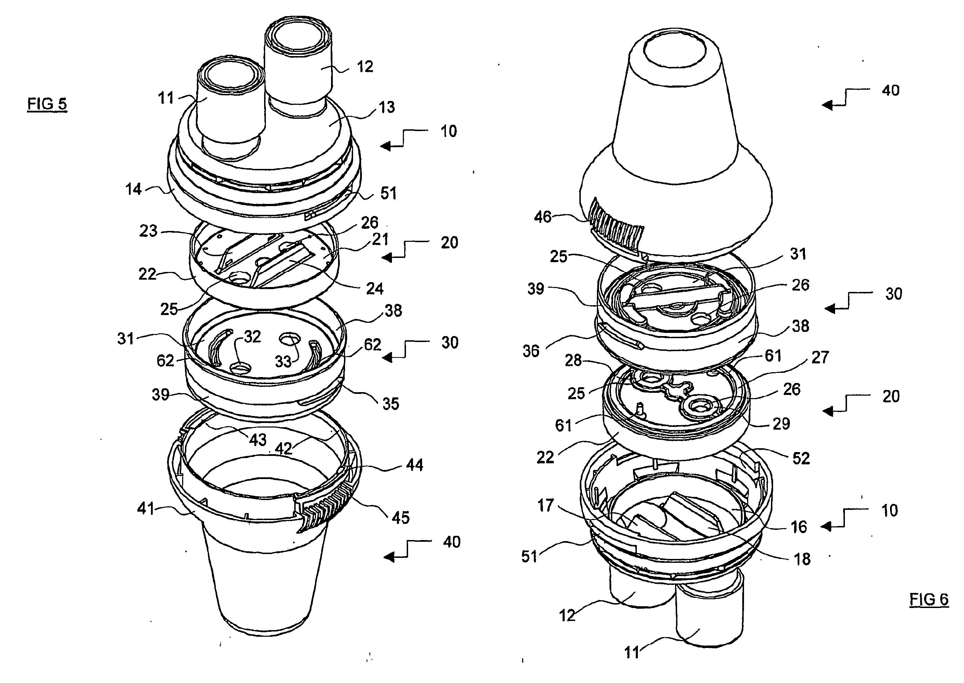

Referring first to Figure 1, a self-sealing water trap according to the invention is generally designated 1 and comprises essentially four components (see Figures 5 and 6). These are a trap top 10, a sealing ring 20, a valve plate 30 and a collecting cup 40. The four components 10,20,30,40 are each moulded in plastics material, the sealing ring 20 being formed in two different materials in a two-shot moulding process, as described below.

The trap top 10 is formed integrally with an inlet 11 and an outlet 12 which, in use, are connected to flexible conduits (not shown) forming part of a mechanical ventilation circuit. The trap top 10 has a generally flat central portion 13 (from which the inlet 11 and outlet 12 extend) and a downwardly depending peripheral skirt 14. An internal annular rib 16 depends downwardly from the underside of the central portion 13, inwardly spaced from the skirt 14, as do a pair of transverse ribs 17,18 situated either side of the lower ends of the inlet 11 and outlet 12.

The sealing ring 20 comprises a circular disc 21 with an upwardly extending peripheral flange 22 and a pair of upwardly extending transverse ribs 23,24. In the region of the disc 21 between the transverse ribs 23,24 there are formed a first pair of openings 25,26. The sealing ring 20 has an interference fit with the trap top 10, the peripheral flange 22 fitting closely, with an interference fit, around the
annular rib 16 of the trap top 10, and the transverse ribs 23,24 having an interference fit with the downwardly depending transverse ribs 17,18 of the trap top 10.

The disc 21 and the flange 22 are moulded in a rigid plastics material. The underside of the disc 21 carries a ring 27 of a thermoset or thermoplastic elastomer (silicone, nitrile etc), this material also forming rings 28,29 around the first pair of openings 25,26. The sealing ring 20 thus comprises a rigid substrate to which the elastomeric material is applied, the component being formed in a two-shot moulding process. Such a process offers manufacturing advantages.

Thus, the trap top 10 and the sealing ring 20 are held in fixed relation and form a substantially enclosed, hollow assembly.

A pair of downwardly (with the trap 1 upright, as shown in Figures 1-5 and 7) projecting location pins 61 are formed integrally with the disc 21 and cooperate with recesses 62 in the valve plate 30, as described below.

The valve plate 30 also comprises a circular disc 31 with a second pair of openings 32,33. The second pair of openings 32,33 are positioned on the disc 31 similarly to the manner in which the first pair of openings 25,26 are positioned on the disc 21 of the sealing ring 20, and are of similar dimension, so that with the valve plate 30 appropriately oriented the first 25,26 and second 32,33 pairs of openings are in registration.

The disc 31 has a peripheral annular flange with upwardly and downwardly extending limbs (38.39 respectively), the disc 31 thus being generally of H-section. The upwardly extending limb 38 has, at its uppermost extent, an outwardly projecting lip 34 which engages a corresponding shoulder on the internal surface of the skirt 14 of the trap top 10 with a snap fit. The valve plate 30 is thus captivated by the trap top 10, the disc 31 being held in abutment with the disc 21 of the sealing ring 20 and in particular with the elastomeric rings 27,28,29. The valve plate 30 is rotatable within the trap top 10. To ensure correct alignment of
the assembly, the location pins 61 are received within arcuate recesses 62 formed in the disc 31.

A pair of part-circumferential lugs 35,36 extend outwardly from the valve plate 30, at diametrically opposed positions.

The collecting cup 40 is of upwardly increasing diameter, the upper part of the cup 40 being of double-walled construction. The outer wall 41 is flared to a diameter matching that of the skirt 14 of the trap top 10. The inner wall 42 projects upwards and is of sufficient diameter to fit between the skirt 14 and the downwardly extending limb 39 of the peripheral flange of the valve plate 30, the inner wall 42 fitting relatively closely about the limb 39.

The inner wall 42 is formed with a pair of diametrically opposed outwardly recessed parts which define recesses 43,44. These recesses 43,44 correspond in shape and dimensions to the lugs 35,36 on the valve plate 30. The recesses 43,44 are undercut such that the recesses 43,44 constitute bayonets which can be received within keeps 51,52 formed in the interior of the skirt 14 of the trap top 10. The external surface of the cup 40 immediately below the bayonets is ridged to form finger grips 45,46.

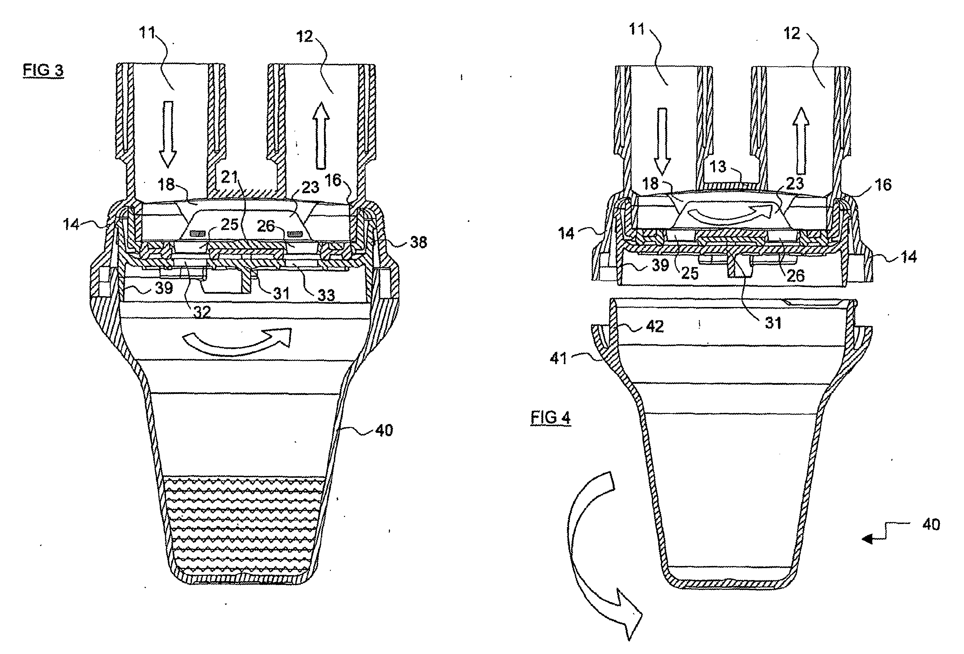

In the normal operating condition, the inlet 11 and outlet 12 of the water trap 1 are connected to respective conduits of a mechanical ventilation circuit. The trap 1 is located substantially at the mid-point of the circuit and at its lowest point. The cup 40 is engaged with the valve plate 30 and held in place by engagement of the bayonets in the keeps 51,52. In this condition, the first 25,26 and second 32,33 pairs of openings are aligned such that air entering the trap 1 via the inlet 11 can also enter the cup 40 before being drawn out through the outlet 12, as indicated by the arrows in Figure 3. Water in the system drains into the trap 1, and moisture carried by the air stream impacts upon the sides of the cup 40 and/or on other exposed internal surfaces of the trap 1, eg the top of the sealing ring 20 and the transverse ribs 23,24, and drains into the cup 40 where it collects.

When it is desired to empty the cup 40, it is simply grasped by means of the finger grips 45,46 and twisted through 90°. This action releases the bayonets from the keeps 51,52 and enables the cup 40 to be removed from the assembly of trap top 10, sealing ring 20 and valve plate 30.

Because the recesses 43,44 receive the lugs 35,36 the valve plate 30 rotates with the cup 40. Thus, rotation of the cup 40 rotates the valve plate 30 in such a manner that the first 25,26 and second 32,33 pairs of openings are no longer in registration and so the passage of air through those openings is closed off. Thus, removal of the cup 40 for emptying automatically seals the trap 1. The integrity of the breathing circuit is thereby maintained, ensuring that the circuit remains airtight during emptying of the cup 40, with no drop in pressure, leakage of fluid or risk of infection by external agents.

When the cup 40 is removed, air can still flow from the inlet 11 to the outlet 12 via the interior of the trap top 10, between the transverse ribs 23,24, as indicated by the arrows in Figure 4.

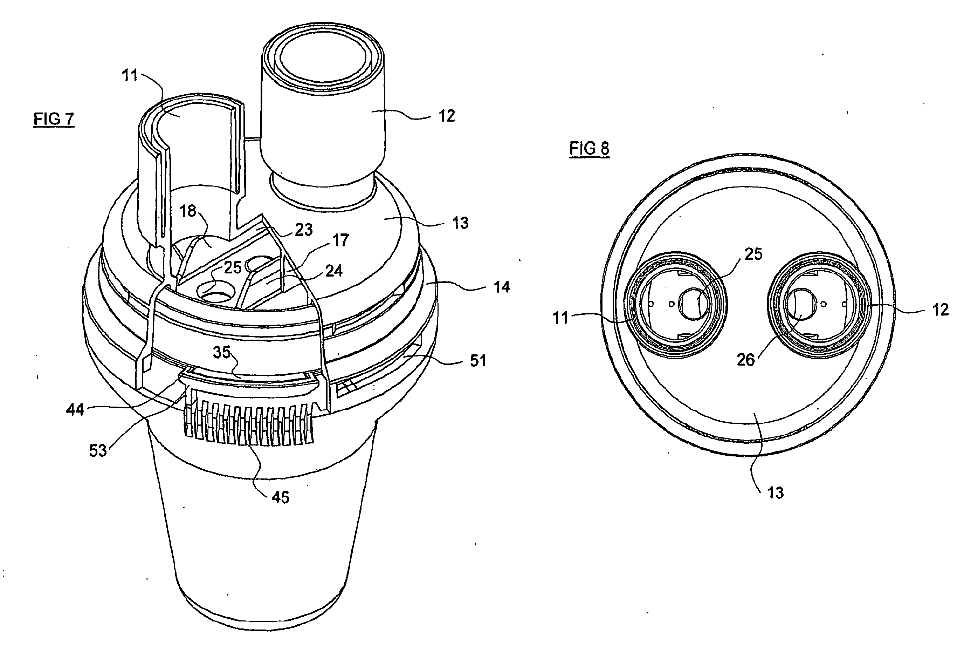

As can be seen in Figure 7 (which shows the cup 40 in a partially disengaged position), the base of the bayonet has a downward projection 53 which constitutes an end-stop. The end-stop engages a corresponding formation on the internal surface of the skirt 14 when the cup 40 is rotated for release from the rest of the trap 1, thereby limiting rotation of the cup 40 and facilitating such release.

As can also best be seen in Figure 7, the lower surface of the keeps 51,52 and the mating faces of the bayonets are shaped to engage with a camming action. This facilitates secure engagement of the cup 40 with the rest of the trap 1, and inhibits unintentional release of the cup 40.

The trap top 10 and the valve plate 30 are most preferably moulded in plastics materials with low coefficients of friction to provide minimal resistance between the components when the water trap 1 is opened and closed.

## Claims

1. A water trap (1) suitable for use in a mechanical ventilation circuit, the water trap (1) comprising
a trap top (10) having formed therein an inlet (11) and an outlet (12) in fluid communication via a chamber, and having a circular base (21) with at least one base aperture (25,26) formed therein,
a rotatable valve plate (30) in abutting relation to said base (21) and rotatable relative thereto, said valve plate (30) having at least one valve aperture (32,33) adapted by rotation of said valve plate (30) to be brought into or out of registration with said base aperture (25,26), and
a fluid collection vessel (40) releasable from the rest of the water trap (1) and operably linked to said valve plate (30) such that, when said fluid collection vessel (40) is engaged with the rest of the water trap (1), said base aperture (25,26) and said valve aperture (32,33) are in registration and said fluid collection vessel (40) is in fluid communication with said trap top (10) and, when said fluid collection vessel (40) is removed from the rest of the water trap (1), said base aperture (25,26) and said valve aperture (32,33) are not in registration and said fluid collection vessel (40) is isolated from said trap top (10), **characterised in that**
the base (21) is moulded in rigid plastics material and an elastomeric seal (27,28,29) is subsequently moulded to the surface of said base (21) that abuts said valve plate (30), the elastomeric seal (27,28,29) comprising a ring of elastomeric material (28,29) around the at least one base aperture (25,26).

2. A water trap (1) as claimed in Claim 1, wherein said elastomeric seal (27,28,29) further comprises a ring of elastomeric material (27) disposed substantially at the periphery of the circular base (21).

3. A water trap (1) as claimed in any preceding claim, wherein the at least one base aperture (25,26) and the at least one valve aperture (32,33) are circular.

4. A water trap (1) as claimed in any preceding claim, wherein there are provided two base apertures (25,26) and two valve apertures (32,33).

5. A water trap (1) as claimed in any preceding claim, wherein the fluid collection vessel (40) engages the trap top (10) with a bayonet-type connection (43,44,51,52).

6. A water trap (1) as claimed in any preceding claim, wherein said trap top (10), said base (21), said valve plate (30) and said fluid collection vessel (40) are all formed in plastics material.

7. A mechanical ventilation circuit including a water trap (1) as claimed in any preceding claim.

8. A mechanical ventilation circuit as claimed in Claim 7, wherein said water trap (1) is positioned substantially at the mid-point of the circuit and at its lowest point.

## Patentansprüche

1. Ein Wasserabscheider (1), der für die Benutzung in einem mechanischen Beatmungskreislauf geeignet ist, wobei der Abscheider (1) umfasst
eine Abscheider-Oberseite (10), die eine darin geformte Einlassöffnung (11) und eine Auslassöffnung (12) besitzt, die über eine Kammer in flüssiger Kommunikation stehen, und die eine kreisförmige Grundfläche (21) mit zumindest einer darin geformten Grundflächen-Öffnung (25, 26) besitzt,
eine drehbare Ventilplatte (30), die in einer aneinander anstoßenden Relation zu der besagten Grundfläche (21) steht und hinsichtlich derer sie drehbar ist, wobei die besagte Ventilplatte (30) mindestens eine Ventilöffnung (32, 33) besitzt, die durch Drehung der besagten Ventilplatte (30) angepasst wird, um in Passung mit der besagten Grundflächen-Öffnung (25, 26) gebracht zu werden, oder aus dieser heraus gebracht zu werden, und
ein Flüssigkeitssammelbehälter (40), der von dem Rest des Wasserabscheiders (1) abtrennbar ist, und in betriebsbereiter Weise mit der besagten Ventilplatte (30) so verbunden ist, dass, wenn der besagte Flüssigkeitssammelbehälter (40) mit dem Rest des Wasserabscheiders (1) verbunden ist, die besagte Grundflächen-Öffnung (25, 26) und die besagte Ventilöffnung (32, 33) sich in Passung befinden, und der besagte Flüssigkeitssammelbehälter (40) mit der besagten Abscheider-Oberseite (10) in flüssiger Kommunikation steht, und, wenn der besagte Flüssigkeitssammelbehälter (40) von dem Rest des Wasserabscheiders (1) entfernt wird, die besagte Grundflächen-Öffnung (25, 26) und die besagte Ventilöffnung (32, 33) sich nicht in Passung befinden, und der besagte Flüssigkeitssammelbehälter (40) von dieser Abscheider-Oberseite (10) isoliert wird, **dadurch gekennzeichnet, dass**
die Grundfläche (21) aus einem starren Kunststoff geformt ist und eine elastomerische Dichtung (27, 28, 29) anschließend auf der Oberfläche dieser Grundfläche (21) geformt wird, die an die besagte Ventilplatte (30) anstößt, wobei die elastomerische Dichtung (27, 28, 29) einen Ring aus elastomerischem Material (28, 29) um die zumindest eine Grundflächen-Öffnung (25, 26) umfasst.

2. Ein Wasserabscheider (1) nach Anspruch 1, wobei die besagte elastomerische Dichtung (27, 28, 29) ferner einen Ring aus elastomerischem Material (27) umfasst, der im Wesentlichen an der Peripherie der kreisförmigen Grundfläche (21) angebracht ist.

3. Ein Wasserabscheider (1) nach einem der vorhergehenden Ansprüche, wobei die zumindest eine Grundflächen-Öffnung (25, 26) und die zumindest eine Ventilöffnung (32, 33) kreisförmig sind.

4. Ein Wasserabscheider (1) nach einem der vorhergehenden Ansprüche, wobei zwei Grundflächen-Öffnungen (25, 26) und zwei Ventilöffnungen (32, 33) vorgesehen sind.

5. Ein Wasserabscheider (1) nach einem der vorhergehenden Ansprüche, wobei der Flüssigkeitssammelbehälter (40) die Abscheider-Oberseite (10) mit einer bajonettartigen Verbindung (43, 44, 51, 52) verbindet.

6. Ein Wasserabscheider (1) nach einem der vorhergehenden Ansprüche, wobei die besagte Abscheider-Oberseite (10), die besagte Grundfläche (21), die besagte Ventilplatte (30) und der besagte Flüssigkeitssammelbehälter (40) allesamt aus Kunststoff hergestellt sind.

7. Ein mechanischer Beatmungskreislauf, der einen Wasserabscheider (1) nach einem der vorhergehenden Ansprüche einschließt.

8. Ein mechanischer Beatmungskreislauf nach Anspruch 7, wobei der besagte Wasserabscheider (1) im Wesentlichen an dem mittleren Punkt des Kreislaufes und an dessen unterstem Punkt positioniert ist.

## Revendications

1. Un collecteur d'eau (1) adapté pour son utilisation dans un circuit de ventilation mécanique, collecteur d'eau (1) qui comprend :
une partie supérieure du collecteur (10) dans laquelle on a disposé une entrée (11) et une sortie (12) qui sont en communication fluide au travers d'une chambre, et qui dispose d'une base circulaire (21) sur laquelle on a aménagé au moins une ouverture de base (25, 26),
une plaque de robinet-vanne (30) qui est en contact avec ladite base (21) et qui peut tourner par rapport à celle-ci, cette plaque de robinet-vanne (30) ayant au moins une ouverture de robinet-vanne (32, 33) qui peut, en tournant ladite plaque de robinet-vanne (30), être alignée avec l'ouverture de ladite base (25, 26) ou bien ne pas être alignée et
un récipient de récupération de fluide (40) qui peut se détacher du reste du collecteur d'eau (1) et qui se trouve uni de façon opérative à ladite plaque de robinet-vanne (30) de telle manière que, quand ledit récipient de récupération de fluide (40) est engrené avec le reste du collecteur d'eau (1), ladite ouverture de base (25, 26) et ladite ouverture de robinet-vanne (32, 33) sont alignées et ledit récipient de récupération de fluide (40) est en communication fluide avec la partie supérieure du collecteur (10) et, quand ledit récipient de récupération de fluide (40) est séparé du reste du collecteur d'eau (1), ladite ouverture de base (25, 26) et ladite ouverture de robinet-vanne (32, 33) ne sont pas alignées et ledit récipient de récupération de fluide (40) est isolé de la partie supérieure du collecteur (10), **caractérisé en ce que**,
la base (21) est moulée en matériel plastique rigide, et postérieurement un joint élastomère est moulé (27, 28, 29) sur la surface de ladite base (21) qui est en contact avec ladite plaque de robinet-vanne (30), le joint élastomère (27, 28, 29) comprenant une bague en matériel élastomère (28, 29) autour d'au moins une ouverture de base (25, 26).

2. Un collecteur d'eau (1) selon la revendication 1, dans lequel ledit joint élastomère (27, 28, 29) comprend également une bague en matériel élastomère (27) disposée substantiellement sur la périphérie de la base circulaire (21).

3. Un collecteur d'eau (1) selon n'importe laquelle des revendications précédentes dans lequel au moins une ouverture de base (25, 26) et au moins une ouverture de robinet-vanne (32, 33) sont circulaires.

4. Un collecteur d'eau (1) selon n'importe laquelle des revendications précédentes dans lequel on a aménagé deux ouvertures de base (25, 26) et deux ouvertures de robinet-vanne (32, 33).

5. Un collecteur d'eau (1) selon n'importe laquelle des revendications précédentes dans lequel le récipient de récupération de fluide (40) engrène la partie supérieure du collecteur (10) avec une connexion type baïonnette (43, 44, 51, 52).

6. Un collecteur d'eau (1) selon n'importe laquelle des revendications précédentes dans lequel ladite partie supérieure du collecteur (10), ladite base (21), ladite plaque de robinet-vanne (30) et ledit récipient de récupération de fluide (40) sont tous faits en matériel plastique.

7. Un circuit de ventilation mécanique qui comprend un collecteur d'eau (1) selon n'importe laquelle des revendications précédentes.

8. Un circuit de ventilation mécanique selon la revendication 7, dans lequel ledit collecteur d'eau (1) est situé substantiellement sur le point intermédiaire du circuit et sur son point le plus bas.
